# EUROPÄISCHE PATENTSCHRIFT

(11) **EP 0 963 246 B1**
(45) Veröffentlichungstag und Bekanntmachung des Hinweises auf die Patenterteilung: **22.10.2003**
(21) Anmeldenummer: 98913553.8
(22) Anmeldetag: 17.02.1998
(51) Int. Cl.: B01J 19/00, C07H 21/00, C07K 1/04

(54) **VORRICHTUNG FÜR EINE AUTOMATISIERTE CHEMISCHE SYNTHESE**
AUTOMATED CHEMICAL SYNTHESIS DEVICE
DISPOSITIF AUTOMATISE DE SYNTHESE CHIMIQUE

(30) Priorität: 17.02.1997 DE 19706089
(43) Veröffentlichungstag der Anmeldung: 15.12.1999
(60) Teilanmeldung: 00117339.2
(73) Patentinhaber: Gesellschaft für Biotechnologische Forschung mbH (GBF), D-38124 Braunschweig (DE); ABIMED Analysen-Technik GmbH, 40736 Langenfeld (DE); IMB Institut für Molekulare Biotechnologie e.V., 07745 Jena (DE)
(72) Erfinder: FRANK, Ronald, D-38124 Braunschweig (DE); MATYSIAK, Stefan, D-38124 Braunschweig (DE); SCHREUER, Olaf, D-38124 Braunschweig (DE); GAUSEPOHL, Heinrich, D-40736 Langenfeld (DE); ROSENTHAL, André, D-07745 Jena (DE)
(74) Vertreter: Boeters, Hans Dietrich, Dr.
(86) Internationale Anmeldenummer: EP9800901
(87) Internationale Veröffentlichungsnummer: WO98035753

(56) Entgegenhaltungen:
- EP-A- 0 529 504
- WO-A-90/02605
- WO-A-94/18226
- WO-A-95/11262
- DE-A- 19 525 258
- US-A- 3 991 627
- US-A- 5 243 540
- JEAN E. RIVER & GARLAND R. MARSHALL (EDS.): "Peptides - Chemistry, Structure and Biology, Proc. 11th Am. Pept. Symp., 9-14 July 1989, La Jolla, CA, USA" 1990 , ESCOM , LEIDEN, NL XP000371822 H. Gausepohl et al.: "Automated multiple peptide synthesis with BOP activation" siehe Seite 1003 - Seite 1004
- STEPHAN HOFFMANN & RONALD FRANK: "A New Safety-Catch Peptide-Resin Linkage for the Direct Release of Peptides into Aqueous Buffers" TETRAHEDRON LETTERS., Bd. 35, Nr. 42, 1994, OXFORD GB, Seiten 7763-7766, XP000653711 in der Anmeldung erwähnt
- ANTHONY V. LEMMO ET AL.: "Characterization of an Inkjet Chemical Microdispenser for Combinatorial Library Synthesis" ANALYTICAL CHEMISTRY., Bd. 69, Nr. 4, 15.Februar 1997, COLUMBUS US, Seiten 543-551, XP000681609

## Beschreibung

Die Synthese von polymeren Biomelekülen, wie Oligonucleotide, Peptide oder unnatürliche Analoge dazu, nach dem Prinzip der Festphasensynthese ist eine etablierte Technik (R. Frank in: GBF, Wissenschaftlicher Ergebnisbericht, (1993) 5-16).

Während multiple parallele Peptidsynthese in offenen Reaktionssystemen zum Stand der Technik gehört (Angew. Chem., 104 (1992) 375-500; Bioorg. Med. Chem. Letters, 3 (1993) 425-430), werden z. B. Oligonucleotide eher einzeln hergestellt. Das Hauptproblem der Oligonucleotidsynthese liegt in der extremen Wasserempfindlichkeit der zum Stand der Technik gehörenden Phosphoramiditchemie (R. Frank loc. cit.). Oligosyntheseautomaten sind daher geschlossene Systeme und arbeiten unter Schutzgas. Ein publiziertes Gerät zur parallelen Synthese von bis zu 96 Oligonucleotiden benutzt ein ebenfalls geschlossenes Reaktionssystem und eine Vielzahl von Ventilen zur Dosierung von Reagenzien (PNAS, 92 (1995) 7912-7915). Hier werden konventionelle Träger und manuelle Prozeduren zur Aufarbeitung verwendet.

WO 94/18 226 betrifft eine Vorrichtung zur automatischen Synthese von Oligonucleotiden unter Verwendung von zweidimensional angeordneten Reaktionssäulen.

WO 95/11 262 betrifft eine Vorrichtung zur parallelen Synthese von Oligonucleotiden unter Verwendung von Arrays mit einer Vielzahl von ventilgesteuerten Reagenzzuführungen.

Dem erfindungsgemäßen Synthesesystem liegt, wie in den Ansprüchen definiert, nun der Gedanke zugrunde, Synthese, Träger, Ankergruppen und Aufarbeitungsprozedur für die simultane vollautomatische Herstellung von Biomolekülen auszulegen. Bei Verteilung der Reagenzien über einen

Pipettierroboter können die Reaktionssäulen in einem für die weitere Bearbeitung geeigneten Format angeordnet werden. Um mit einem Pipettierroboter auch ein wasser- und luftempfindliches Syntheseprotokoll durchführen zu können, müssen bestimmte konstruktive Maßnahmen ergriffen werden. Beispielhaft für eine mögliche Lösung werden im folgenden das Funktionsprinzip des Automaten und der Ablauf einer Synthese beschrieben.

Der Automat kann mit konventionellen Trägern und Reagenzien arbeiten.

Durch Anschluß eines speziellen Verfahrens für simultane Reinigung und Aliquotierung wird die Qualität der Produkte verbessert und die Verwendung vereinfacht.

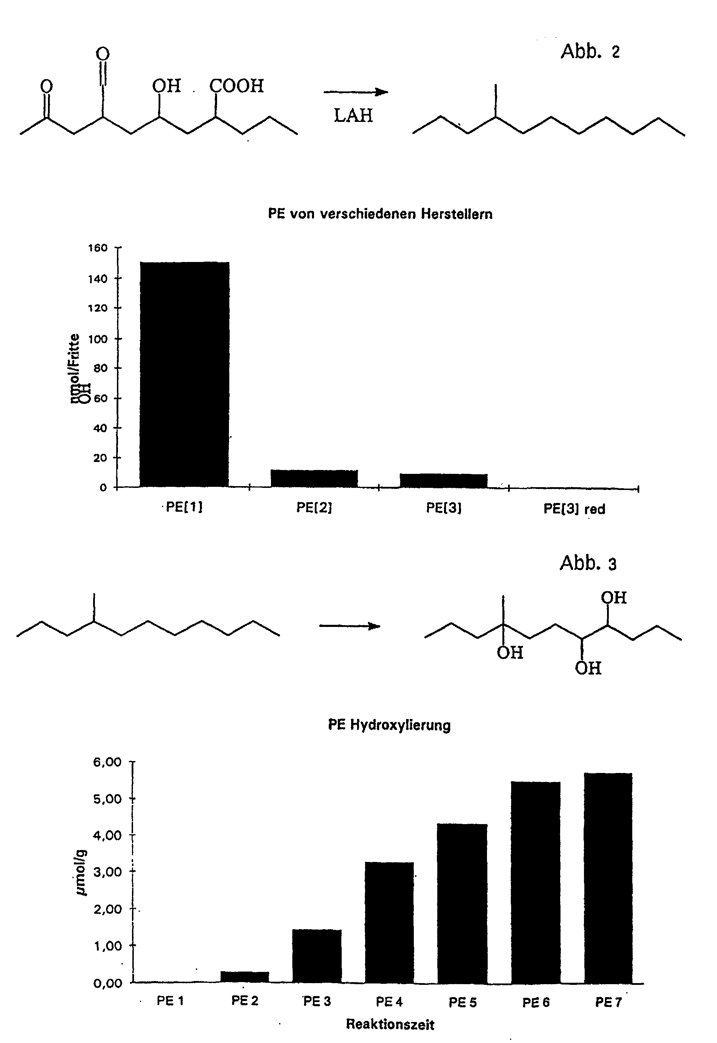

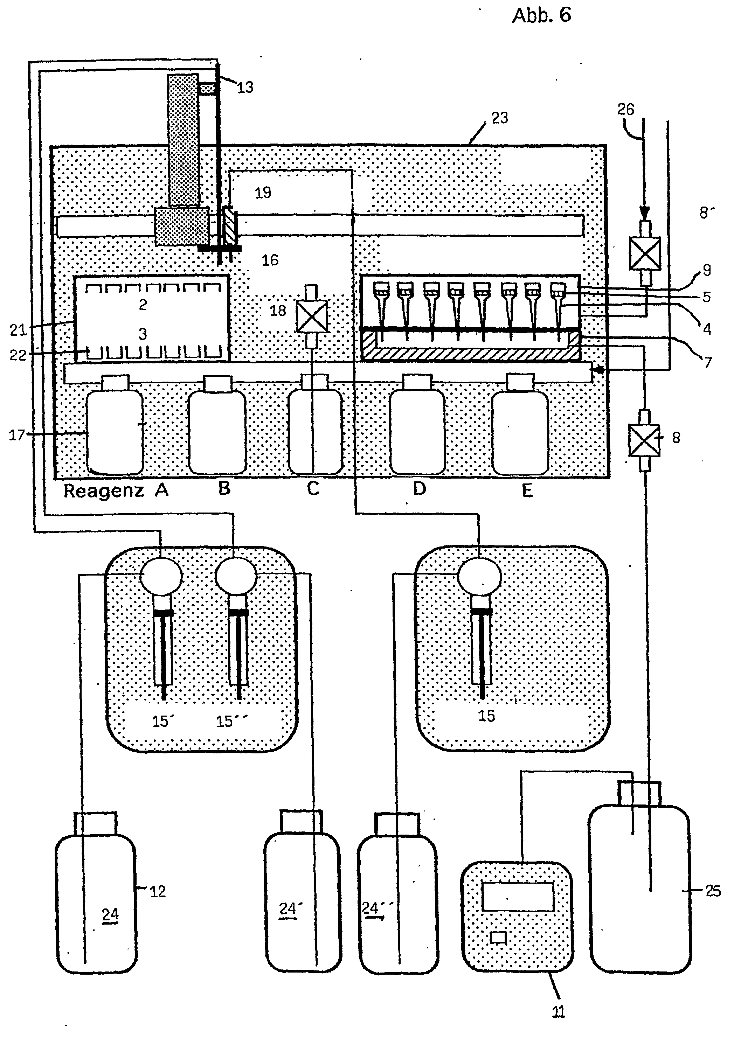

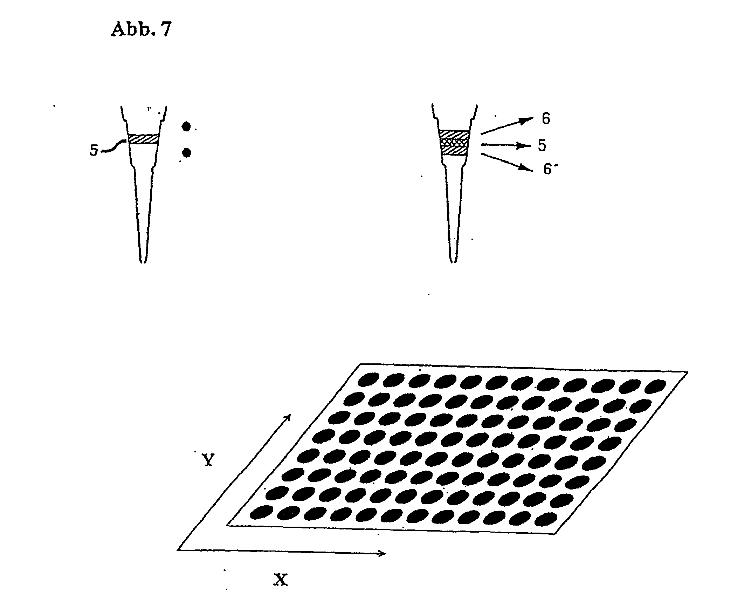

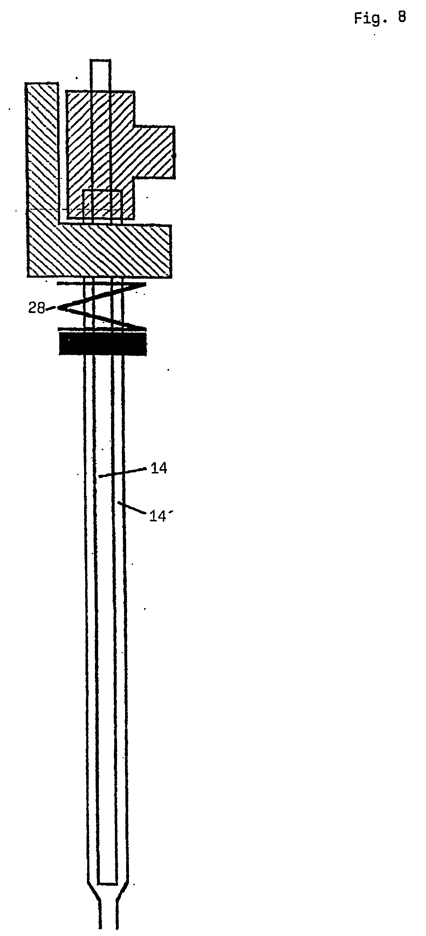

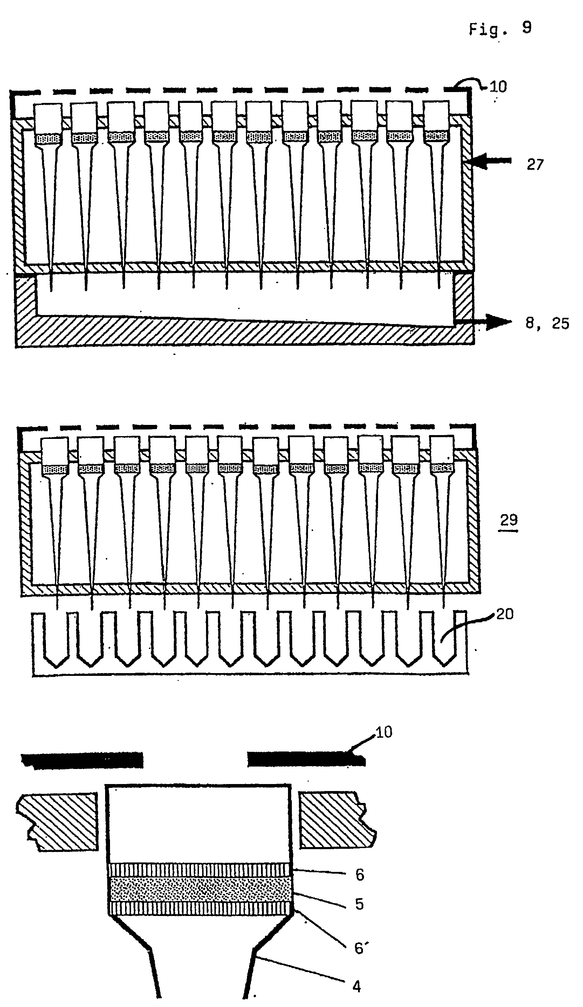

Insbesondere betrifft die Erfindung folgende Ausführungsformen: Vorrichtung für eine automatisierte simultane chemische Synthese und Aufreinigung einer Vielzahl von Produkten an der Festphase sowie Trägermaterial und chemische Bausteine für die Festphasensynthese, dadurch gekennzeichnet, daß
1. Eine Vielzahl (10 bis 1000, vorzugsweise 48 und ein Vielfaches davon, vorzugsweise 400) von separaten, nach oben und unten offenen Reaktionsgefäßen bzw. Reaktoren 4 als parallel in einem Block 9 (Abb. 6) angeordnete Kanäle oder kleine Säulen vorgesehen werden, die entweder gemeinsam oder einzeln entnehmbar sind; daß in den Kanälen/Säulen das Trägermaterial 5 beispielsweise konventionelles Trägermaterial wie Zellulosepapiere für die Synthese (Festphase) entweder zwischen zwei inerte poröse Frittenböden 6,6'(beispielsweise aus Polyolefinen wie PE, PP oder PTFE) oder vorzugsweise selbst als chemisch modifizerter Fritten- oder Filterboden eingebracht wird (Abb. 7), so daß von oben zugegebene flüssige Medien allein durch die Oberflächenspannung und Benetzung des Materials im Reaktor 4 festgehalten werden;
2. daß der Reaktorblock 9 der Reaktoren 4 nach 1. auf eine Wanne 7 aufgesetzt wird, die über ein schaltbares Ventil 8 an eine Vakuumpumpe 11 angeschlossen ist und so die flüssigen Medien aus den Reaktoren 4 und den darin enthaltenen Trägermaterialien 5 simultan abgesaugt werden können; gegebenenfalls kann eine Abfallflasche 25 zwischen Ventil 8 und Vakuumpumpe 11 vorgesehen sein;
3. daß die oberen Eingänge der Säulen der Säulen der Reaktionsgefäße 4 im Reaktorblock bzw. Reaktionsblock 9 nach 1. abgedeckt durch eine darüber angebrachte Lochblende 10 in Figur 9 (Prallblech) mit Inertgas 27 (z.B. Sticksoff 26, Argon) geflutet werden können und der Inertgasstrom gegebenenfalls während des Absaugvorganges nach 2. deutlich erhöht wird; alternativ, der Raum über den Säulen, Kanälen der Reaktionsgefäße 4 durch eine zweite, verschiebbare Lochblende gezielt verschlossen werden kann, um die Reagenzien mit Inertgasüberdruck aus den Säulen, Kanälen der Reaktionsgefäße 4 auszublasen;
4. daß chemische Bausteine 3, Reagenzien 2 und Lösungsmittel 24, 24', 24'', von einem xyz-Pippettierroboter 23 über elektronisch steuerbare Dosierspritzen 15, 15', 15''(Dilutoren) mit einer oder mehreren Dosiernadeln 13 und gegebenenfalls zusätzlich einem oder mehreren Dosierkämmen 19 auf die Reaktionsgefäße 4 verteilt werden, so daß jeder Reaktor 4 einzeln addressierbar ist;
5. daß die Dosiernadel 13 nach 4. mit mehreren (mindestens zwei; Innenkanüle 14, Außenkanüle 14') an separate Dosierspitzen 15, 15', 15'' angeschlossenen, also getrennt befüllbaren inneren Kanälen ausgerüstet ist, deren Enden sich erst vor dem Auslaß treffen (Abb. 8) und so bei simultaner Dosierung mehrerer Reagenzien 3 die Mischung erst kurz vor der Abgabe in der Spitze der Dosiernadel 13 erfolgt, wobei ein Kanal auch an die Inertgasversorgung angeschlossen sein kann und so über ein Inertgaspuls das Mischvolumen ausgedrückt werden kann;
6. daß die Dosiernadel 13 nach 4. und 5. in der Längsachse federnd (Feder 28) gelagert ist (Figur 8), um ohne Beschädigung auf das Trägermaterial 5 oder die Deckfritten 6, 6'in den Kanälen der Reaktionsgefäße 4 aufsetzen zu können und so auch kleinste Volumina bis herunter zu 1 Nanoliter sicher absetzen zu können;
7. daß eine Vielzahl (zwei bis einhundert, vorzugsweise 24) an chemisch Bausteinen 3 und Reagenzien 2, gegebenenfalls in geeigneten Lösungsmitteln gelöst, in mit Septen verschlossenen Gefäßen 22, die in einem vom Reaktionsblock 9 getrennten Reagenzienblock 21 angeordnet sind, bereitgestellt werden;
8. daß die mit Septen verschlossenen Hälse der Gefäße 22 im Reagenzienblock 21 nach 7. abgedeckt durch eine darüber angebrachte Lochblende (Prallblech) mit Inertgas (z.B. Stickstoff, Argon) geflutet werden können;
9. daß Reagenzien mit der Dosiernadel 13 nach 4. und 5. auch aus Transferports 16 entnommen werden können, die entweder direkt oder über schaltbare Ventile 18 mit Vorratsflaschen 17 verbunden sind **(Abb. 6)** und diese Vorratsflaschen 17 unter einem geringen Überdruck an Inertgas stehen;
10. daß Lösungsmittel und Reagenzien aus Solventflaschen 12 mittels Dosierspritzen 15 oder durch Inertgasüberdruck auch über einen oder mehrere Dosierkämme 19 nach 4. simultan auf mehrere Reaktoren 4 reihenweise verteilt werden können;
11. daß das Trägermaterial 5 nach 1. eine Schicht im Reaktorkanal bildet, die gleichmäßig von den oben aufgegebenen Reagenzien und Lösungsmitteln allein durch die Schwerkraft durchströmt wird. Nach dem zum Stand der Technik gehörenden Prinzip der Festphasensynthese **(Abb. 2)** werden so die einzelnen Produkte je Reaktor 4 kovalent fixiert auf der Oberfläche des Trägermaterials 5 und parallel durch eine Abfolge von Pipettieroperationen schrittweise aufgebaut. Während aller Syntheseschritte (Aufbaureaktionen, repetitive Schutzgruppenabspaltungen und Waschoperationen) bleiben die Produkte am Trägermaterial 5 kovalent verknüpft und werden erst in einem oder mehreren letzten Reaktionsschritten vom Trägermaterial 5 abgespalten und in Lösung gebracht;
12. daß die chemischen Bausteine 5 (Monomere), die für den Aufbau der Produkte eingesetzt werden, als ASCII-Lettern codiert sind und die Produkte so als Abfolge von Aufbaureaktionen (Monomereinbaureaktionen) durch ASCII-Worte beschrieben sind; die Gesamtheit aller Produkte für ein Syntheseprogramm ist somit eine Liste von ASCII-Worten, die von einer geeigneten Software auf einem Steuerungsrechner in Ventilschaltung-, Dosierspritzenbewegungs- und Roboterarmbewegungsoperationen umgesetzt wird, wobei jeder Monomereinbau aus einer Folge von mehreren Reaktionsschritten und Schaltungsoperationen bestehen kann;
13. daß für die kovalente Verknüpfung der Produkte mit dem Trägermaterial 5 nach 11. geeignete Verknüpfungsbausteine (Linker) vorgesehen werden, die eine selektive und schonende finale Abspaltung der Produkte erlauben;
14. daß die Zielprodukte der Synthesen mit einer als Affinitätslabel nutzbaren Gruppe versehen sind, über die die Zielprodukte an eine entsprechende Affinitätsphase gebunden werden können. Damit werden die aus den Reaktoren 4 eluierten Produkte in eine der Reaktoranordnung komplementären Anordnung 29 von Affinitätssäulen **(Abb. 9)** gereinigt. Nachfolgend werden die Zielprodukte durch einfache automatisierte Waschoder Abspaltungsoperationen aus den Affinitätssäulen in eine der Reaktoranordnung komplementären Anordnung von Auffanggefäßen 20 in Figur 9 eluiert;
15. daß die Bindungskapazitäten der Affinitätssäulen nach 14. so limitiert sind, daß auch bei unterschiedlichen Syntheseausbeuten je Reaktor 4 eine gleiche Mindestmenge an Zielprodukt gebunden und eluiert wird, damit alle Produkte einer multiplen Synthese in äquimolaren Mengen erhalten werden.

Auf der Arbeitsfläche eines beispielhaften Pipettierroboters sind angeordnet:
- Ständer für Derivatelösungen, evtl. unter Schutzgas mit Septum
- Entnahmeports für Reagenzien, hier durch Ventile schaltbar
- Reagenzienflaschen unter Schutzgas
- Halterung für Reaktionssäulen

Die Reaktionssäulen bestehen aus Kunststoffröhrchen mit eingesetzten Fritten, die den eigentlichen Syntheseträger einschließen, den Träger in einer definierten Position fixieren oder selbst als Träger derivatisiert wurden. Als Röhrchen wurden hier handelsübliche Pipettenspitzen verwendet. Alternativ ist die Verwendung eines zusammenhängenden Spritzgußteils möglich, das einzelne Kavitäten mit Filterfritten enthält (z.B. von PolyFiltronics, Rockland, MA, USA, erhältlich).

Das Reaktionssystem kann im oberen Teil mit Schutzgas gespült werden. Eine Lochblende als Abdeckung kann auch bei kleinem Volumenstrom das Eindringen von Luft verhindern.

Der untere Teil des Reaktionssystems kann zur Absaugung der Reagenzien mit Vakuum beaufschlagt werden. In einer alternativen Anordnung kommt eine zweite, verschiebbare Lochblende zur Anwendung. Damit lassen sich die Löcher bei Bedarf verschließen und die Reaktionssäulen durch einen Überdruck von Schutzgas ausblasen.

Zur Dosierung von Reagenzien ist der Roboter mit einer Kanüle versehen, die mindestens zwei unabhängige Kanäle aufweist. Diese Kanäle treffen sich erst sehr kurz vor dem Auslaß. Über angeschlossene motorbetriebene Dosierspritzen lassen sich Reagenzien separat aufnehmen und simultan abgeben. Im einfachsten Fall besteht die Kanüle aus zwei konzentrisch angeordneten Rohren (vgl. Abbildung). Die Kanüle ist in der Längsachse federnd gelagert, um auch kleinste Volumina bis herunter zu 1 µl ohne Beschädigung der Trägerfritten auf diesen absetzen zu können.

Um die Synthesegeschwindigkeit zu erhöhen, kann eines oder mehrere der Reagenzien und Lösungsmittel auch über einen Verteilerkamm dosiert werden. Dieser Verteilerkamm wird entweder auch über eine Dosierspritze bedient, oder er ist über Ventile mit einem oder mehreren Vorratsbehältern unter Überdruck anschließbar. Der Verteilerkamm dosiert Reagenzien immer simultan auf eine Reihe von Reaktionssäulen. Die Synthese besteht nun aus einer Reihe von programmgesteuerten Übertragungen von Reagenzien und Lösungsmitteln aus den Vorratsbehältern in die Reaktionssäulen. Syntheseablauf und Art der Reagenzien sind im Prinzip bekannt und Stand der Technik.

Die Synthese beginnt mit der Festlegung der Sequenzen als Liste von ASCII-Zeichenketten im steuernden Computerprogramm. Der Syntheseablauf wird als Abfolge von Arbeitsschritten mit Angabe der Reagenzien, der jeweils zu übertragenden Volumina und der einzuhaltenden Reaktionszeiten definiert.

Das Gerät wird dann mit den notwendigen Reagenzien und Syntheseträgern gestückt. Konventionelle Träger nach dem Stand der Technik erfordern die Zuordnung zu den einzelnen Sequenzen, da der erste Baustein außerhalb des Geräts separat aufgebracht werden muß.

Die Synthese beginnt typischerweise mit einem Waschschritt, der vorzugsweise über den Verteilerkamm ausgeführt wird. Zum Entfernen der Lösungsmittel wird das Absaugventil für eine bestimmte Zeit geschaltet und der untere Teil des Reaktionssystems mit Vakuum beaufschlagt. In dieser Zeit wird der Schutzgasstrom zum oberen Teil deutlich erhöht, um das Eindringen von Fremdluft weitgehend zu verhindern. Anschließend wird typischerweise eine Lösung zur Abspaltung der temporären Schutzgruppen über die Kanüle verteilt, z.B. Trichloressigsäure in Acetonitril. Die Kanüle wird zunächst in den geschlossenen Transferport gefahren, der gegen die Außenseite der Kanüle dicht abschließt. Dann wird das Ventil geöffnet und das Reagenz in einen der Kanäle der Kanüle aufgezogen. In der Regel wird mehr als die benötigte Menge aufgezogen, da es im Genzbereich im Schlauch zwischen Kanüle und Dilutor zu Vermischungen kommen kann. Der Grenzbereich zwischen den Flüssigkeiten wird in der Regel durch eine zusätzlich aufgezogene Luftblase definiert. Das Ventil wird wieder geschlossen, und die Kanüle fährt die erste Reaktionssäule an. Die Kanüle wird gegebenenfalls auf der oberen Fritte oder dem Träger selbst aufgesetzt und das erste Aliquot des Reagenzes abgegeben. Die weiteren Positionen werden analog angefahren.

Anschließend wird der Überschuß im Schlauch verworfen und die Kanüle gespült.

Bei Reagenzien, die gemischt werden müssen, z.B. zur Aktivierung der Monomerbausteine, wird zunächst wie beschrieben eines der Reagenzien aufgenommen. Anschließend wird das zweite (und gegebenenfalls weitere) Reagenz in den zweiten Kanal der Kanüle aufgesaugt. Um schon für die erste Dosierung eine korrekte Mischung zu liefern, wird ein Teil des Überschusses zu Beginn durch gleichzeitiges Betätigen beider Dilutorspritzen verworfen. Die Kanüle fährt dann auf die vom Programm vorgegebenen Positionen, setzt auf der Fritte auf und dosiert das Reaktionsgemisch durch gleichzeitiges Betätigen der Dilutorspritzen. Dieses Verfahren ist der entscheidende Schritt der Synthese: Durch die Trennung der Reagenzien bis zur Dosierung auf den Träger wird die Konkurrenzreaktion zur Kupplung, die Hydrolyse durch Spuren von Wasser, verzögert. Damit ist die Synthese in einem eigentlich zur Atmosphäre offenen Reaktionssystem entgegen der vorherrschenden Meinung von Fachleuten doch möglich.

Der weitere Syntheseablauf besteht aus einer Abfolge ähnlicher Schritte mit wechselnden, vom Programmablauf festgelegten Reagenzien.

Im Anschluß an die Synthese erfolgt die bekannte Entschützung und Abspaltung der Produkte vom Träger, z.B. durch Inkubation mit konzentrierter Ammoniaklösung.

Die Aufarbeitung wird ebenfalls erleichtert durch die Anorndung der Reaktionsgefäße in einem Standardformat, z.B. dem von Mikrotiterplatten.

Da die Reaktionen während der Synthese niemals vollständig ablaufen, ist es durchaus üblich, wenn auch nicht immer notwendig, die Produkte zu reinigen. Hier bietet sich das in EP 0 174 525 beschriebene Verfahren an. Alternativ lassen sich auch andere bekannte Affinitätsreinigungen anwenden, z.B. über die Interaktion von Biotin und Avidin (Biochem, J., 316 (1996) 193-199; Peptide Res., 2 (1989) 189-194).

Die meisten Anwendungen erfordern eine relativ genau bestimmte Menge an Syntheseprodukt. Im Fall der Oligonucleotide für Sequenzierreaktionen ist das nur ein Bruchteil der Syntheseausbeute. Hier läßt sich die Ausbeute über alle Syntheseansätze erfindungsgemäß durch Verwendung einer limitierenden Menge an Affinitätsmatrix nivellieren. Die Menge an Affinitätsmatrix wird gut definiert und in jedem Fall deutlich mehr markiertes Syntheseprodukt aufgegeben als der Bindungskapazität entspricht.

Die anschließend eluierten Mengen an Syntheseprodukt sind dann in etwa gleich . Die Kombination von Affinitätsreinigung und Aliquotierung ist neu und erspart aufwendige Konzentrationsmessungen.

Durch den modularen Aufbau des Syntheseautomaten wird es möglich, Synthese, Abspaltung, Reinigung und Aliquotierung in einem Gerät zu vereinen. Bei Anwendung des Verfahrens nach EP 0 174 525 kann ein Affinitätsträger aus Polymermaterial, z.B. Polystyrol, Polyethylen oder Modifikationen davon, benetzt werden. Das Material kann so gewählt werden, daß es gegenüber der Synthese von Schutzgruppenabspaltung inert ist. Es läßt sich dann sogar als Fritte unter dem Syntheseträger anordnen, was die Aufarbeitung gegenüber dem Stand der Technik erheblich vereinfachen würde.

Nachstehend wird die Erfindung anhand von Abbildungen noch näher erläutert. Es zeigen:
Abbildung 1A: Molekulare Moldule für die Festphasensynthese von Oligonucleotiden. P = polymeres Trägermaterial; X = chemische Funktion zur Verankerung (-O- oder -NH-); Spacer = Abstandhalter; Linker = Einheit zur reversiblen Verankerung des 1. Nucleotidbausteins; N_{1,2}= Nucleotidbausteine
Abbildung 2: Reaktivität der Oberflächen verschiedener PE-Materialien im Tritylierungs-/Detritylierungstest; red. = reduktiv gesäubert
Abbildung 3: Gesteuerte Hydroxylierung von PE-Material
Abbildung 4: Beispiele für die in Abbildung 1 konzipierten modularen Syntheseträger. Ausgangsmaterial ist hydroxyliertes Polymer; 1 und 2 sind konventionelle Trägerbeladungen mit 3'-Nucleosidsuccinaten ohne und mit Spacer;
Abbildung 5: Beispiel für die Qualitäten von Oligonucleotid-Syntheseprodukten, hergestellt in einem konventionellen Synthesizer auf konventionellem Trägerharz (oben) und auf PE-Frittenmaterial derivatisiert nach Abb. 4, Typ 1
   Sequenz: decameres Thymidylat ("Tr-off"), voll entschützt;
   Automat: Phamacia Gene Assembler;
   Trägermaterial: Phamacia T-Träger (CPG);
   Linker: Succin
   Synthese: optimiert

   Sequenz: decameres Thymidylat (Tr-off"), voll entschützt;
   Automat: Pharmacia Gene Assembler;
   Trägermaterial: funktionalisierte PE-Fritten (IRIS 45);
   Linker: Succinat;
   Synthese: nicht optimiert;
Abbildung 6: erfindungsgemäße Vorrichtung
Abbildung 7: Reaktoren und Reaktionsanordnung im 8 x 12 Raster
Abbildung 8: Doppelkanüle mit Federbelastung
Abbildung 9: Reaktormodul im 8 x 12 Raster

### A) Chemische Voraussetzungen zur hochparallelen DNA-Synthese

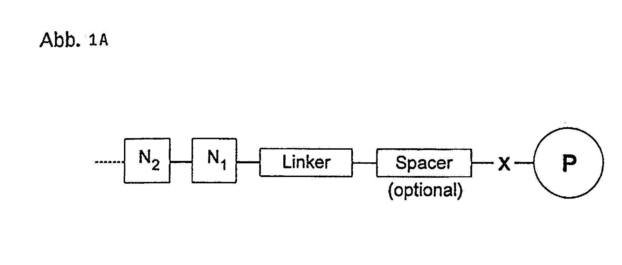

Das Arbeitsprinzip des konzipierten multiplen Syntheseautomaten sieht ein Raster von einfachen kleinen Reaktoren vor, in denen kleine poröse Membranfritten das Trägermaterial für die Synthese darstellen. Der Synthesemaßstab je Reaktor soll an die Anwendung als Sequenzier-Primer optimal angepaßt sein und im Bereich 1 bis 10 Nanomol liegen. Das Konzept zur chemischen Derivatisierung des Trägermaterials für den Einsatz zum schrittweisen Aufbau von Oligonucleotiden ist in **Abb. 1A** dargestellt.

Es wurden zunächst ausreichend empfindliche Detektionsreaktionen etabliert, um die einzelnen Derivatisierungsschritte quantitativ im Nanomol-Maßstab verfolgen zu können:
- für Hydroxyfunktionen: Tritylierung mit Dimethoxytritylchlorid in Pyridin gefolgt von der sauren Detritylierung mit Dichloressigsäure in Dichlorethan und photometrische Bestimmung des Dimethoxytritylkations;
- für Aminofunktionen: Anfärbung mit dem anionischen Farbstoff Bromphenolblau in Dimethylformamid, gefolgt von der basischen Dissoziation und photometrischen Bestimmung des Bromphenolblau-Anions.

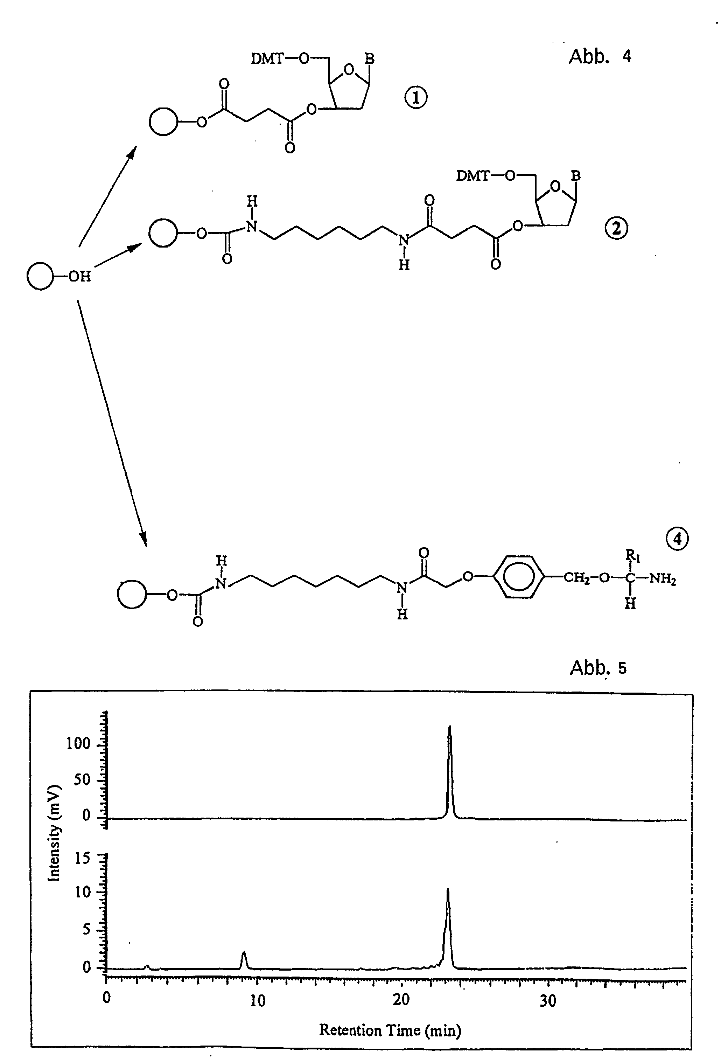

Verschiedenste, kommerziell erhältliche Membranmaterialien auf Polyolefinbasis wurden dann auf Eignung untersucht. Je nach Herstellung weisen diese Materialen schon eine unspezifische oxidative Alterung der Oberfläche mit Hydroxyl-, Keton-, Aldehyd- und Carboxylgruppen auf **(Abb. 2)**. Daher wurde eine Prozedur entwickelt, die diese Polymeroberfläche zunächst reduktiv "säubert" und anschließend durch selektive und leicht steuerbare Oxidation ausschließlich die hier gewünschten Hydroxylgruppen generiert **(Abb. 3).** Damit kann jetzt Frittenmaterial mit vorbestimmter und für den Einsatz zur Oligonucleotidsynthese geeigneter Funktionalität hergestellt werden. Eine Vielzahl von chemischen Umsetzungen dieser sowie auch anderer hydroxylierter Materialien (z.B. Cellulosepapiere) wurde durchgeführt, um geeignete "Spacer" und "Linker" aufzubringen **(Abb. 4).** Die so erhaltenen Fritten wurden in einem kommerziellen .Syntheseautomaten für Oligonucleotidsynthesen erfolgreich eingesetzt **(Abb. 5)** und stehen jetzt für Tests im Reaktormodul des neuen Automaten zur Verfügung.

Idealerweise sollte das nach Abbildung 1A vorbereitete Trägermaterial (Träger-Spacer-Linker) universell für jede Oligonucleotidsequenz einsetzbar sein, so daß keine individuelle Konfigurierung des Syntheserasters notwendig ist. Dies ist durch die konventionelle Beladung in einer separaten Reaktion mit 3'-Nucleosidsuccinaten nicht gegeben (jede Sequenz erfordert einen der vier verschiedenen beladenen Träger).

### B) Experimentalversion einer DNA-Synthesemaschine

Eine erste Version des Gerätes wurde anhand des ursprünglichen Konzeptes aufgebaut und die notwendige Steuerungssoftware extern erstellt **(Abb. 6 bis 9).**

### C) Beispiele für Quantifizierung (und Reinigung) der produzierten Oligonucleotide

Je nach Basensequenz der synthetisierten Oligonucleotide erhält man unterschiedliche Gesamtausbeuten. Das folgende Verfahren ermöglicht es, eine einheitliche definierte Menge der verschiedenen Oligonucleotide zu erhalten. Gleichzeitig werden die Oligonucleotide dabei gereinigt.
1. Als Material für die Quantifizierung dient ein hydrophobes Polyolefin-Pulver (PE, PP oder PTFE). Definierte Mengen dieses Pulvers werden in Sterilfilter-Spitzen (Fa. Eppendorf) oder in entsprechenden Spitzen mit Fritten gegeben (z.B. 20 mg, 10 mg, 5 mg oder 2,5 mg). Vorbereitet wird das Pulver durch Waschen mit Acetonitril (je 3 x 250 µl) und anschließend mit 1 M Triethylaminacetat-Puffer (TEAA) (je 3 x 250 µl).
2. Die mit dem PRIME96-Syntheseroboter dargestellten Oligonucleotide, die noch die hydrophobe Trityl-Schutzgruppe besitzen, werden vom Träger abgespalten. Man erhält so die Lösung A (in diesem Beispiel: 200 µl 33% NH₃-Lösung). Diese Lösung A wird 1:1 mit Wasser verdünnt und auf die vorbereiteten Säulen gegeben und langsam mit Hilfe einer Spritze durchgedrückt.
3. Es wird mit 2,5% NH₃ (aq) (je 3 x 250 µl) und Wasser (je 3 x 250 µl) gespült.
4. Die Trityl-Schutzgruppe wird nun mit 2% Trifluoressigsäure (aq) (je 3 x 250 µl) abgespalten. Nach 5 min wird mit Wasser (je 3 x 250 µl) gespült.
5. Eluiert wird da quantifizierte und gereinigte Oligonucleotid mit 20% Acetonitril (aq) (je 3 x 250 µl).

### Beispiel 1:

### Aufgetragen wurden 7,2 OD (ca. 40 nMol) eines gemischten 18'mers

| mg Quantifizierungs-Harz* | Auftragseluat (OD₂₆₀) | TFA-Eluat (OD₂₆₀) | End-produkt (OD₂₆₀) | Wiederfindungsrate (%) |
|---|---|---|---|---|
| 2,5 | 5,5 | 0,9 | 0,427 | 94 |
| 5 | 5,22 | 0,9 | 0,968 | 98 |
| 10 | 4,3 | 0,7 | 1,93 | 95 |
| 20 | 3,32 | 0,4 | 2,68 | 88 |

| | | | | |
|---|---|---|---|---|
| • Micropure, BTI Technologies, San Raffael, CA, USA Als "Faustformel" ergibt sich aus dieser Testreihe, daß ca. 1 nMol Oligonucleotid pro mg Quantifizierungsharz gebunden wird. Die Quantifizierung beruht darauf, daß eine definierte Menge des zunächst noch tritylierten Oligonucleotids pro mg des eingesetzten Reinigungsmaterials gebunden wird. Da die Menge des mit dem PRIME96 jeweils sythetisierten Oligonucleotids stets größer ist als die Menge, die vom Reinigungsmaterial gebunden werden kann, ist so auf einfache Weise eine Quantifizierung möglich. | | | | |

### D) Beispiel für Trägermaterial für die Festphasensynthese mit einem Syntheseroboter

### Innovatives Konzept:

Unser innovatives Synthesekonzept basiert darauf, daß die verschiedenen Reaktionslösungen allein durch Kapillarkräfte an einem festen Träger haften und mit diesem reagieren können. Dabei kann also so auf anfällige und teure Ventilsysteme verzichtet werden.

Dieses kann zum einen dadurch realisiert werden, daß man zwischen zwei inerte Fritten ein konventionelles Trägermaterial einbringt. Noch besser ist es jedoch, einen Träger zu entwikkeln, der bereits eine definierte poröse Struktur besitzt, um diesen dann zu funktionalisieren. Das letztere Verfahren bietet dabei folgende Vorteile:
- kommerziell erhältliches Trägermaterial mit definiertem macroporösem Porendurchmesser
- reproduzierbare Herstellung
- große Oberfläche
- gute Durchströmungseigenschaften
- definierte Struktur
- mechanisch stabil
- chemisch weitgehend inertes Grundmaterial, nur die Oberfläche wird derivatisiert

Ein derartiges neues Trägersystem haben wir hier entwickelt, wobei besonders die poröse Struktur des Ausgangsmaterials eine Funktionalisierung mit bekannten Verfahren ausschloß.

Funktionalisierung von Polyolefin-Fritten (am Beispiel von Polyethylen- und Polypropylen)

### 1) Reduktion

Bedingt durch den Herstellungsprozeß (Sinterung) sind die kommerziell erhältlichen Polyolefin-Fritten bereits oxidiert (Carboxy-, Carbonyl-, Hydroxylgruppen). Um hier ein einheitliches Startmaterial herzustellen, ist zunächst eine Reduktion nötig. Dazu werden in einem Dreihalskolben mit Rückflußkühler 50 mMol LiAlH₄ vorgelegt und 50 ml trockener Diethylether zugetropft. Danach werden 1 g PE-Fritten hinzugefügt und 6 h unter Rückfluß erhitzt. Hiernach wird zunächst mit feuchtem Ether der Überschuß von LiAlH₄ hydrolysiert und der Niederschlag mit 10% H₂SO₄ wieder aufgelöst. Es wird 3 x mit 100 ml Wasser, dann mit 2 x 100 ml 10% NaHCO₃, danach wieder mit 3 x 100 ml Wasser und 1 x 100 ml MeOH gewaschen und im HV getrocknet.

### 2) Direkte Hydroxylierung

1 g der reduzierten Polyolefin-Fritten werden zu einer Lösung von 10 ml H₂O₂ (30% aq) und 100 ml Trifluoressigsäure gegeben. Unter Rückfluß wird 15 bis 60 min erhitzt.
Je nach Reaktionszeit ergeben sich Beladungen von 250 nMol OH/g (15 min) bis 5 µMol OH/g (60 min). (Die Beladung mit OH-Gruppen wurde über DMT-Kopplung bestimmt.)
Die hier verwendete Methode zur Hydroxylierung ist in der Literatur bis jetzt nur für Alkane und Cycloalkane beschrieben.

Die so hergestellten porösen hydroxylierten Träger sind universell für alle Festphasensynthesen einsetzbar.

### 3.) Kopplung des ersten Bausteins (Startnucleosids) an den Träger

### Beispiel 1 (s.a. Abb. 4) :

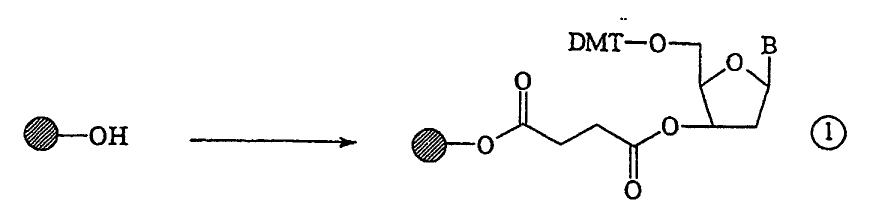

20 µMol DMT-dT^{an}-Succ werden in 100 µl DMF gelöst und 31 µMol N,N'-Diisopropylcarbodiimid (DICD) zugemischt. Nach 10 min werden 25 µMol Methylimidazol zugemischt und 60 mg der hydroxylierten Polyolefin-Fritten (Darstellung s.o.) unter Stickstoff zugegeben. Nach 24 Stunden bei 25°C werden die Fritten nacheinander mit DMF, Pyridin und Methylenchlorid gewaschen.

### Beispiel 2: Einführung eines Spacers (s.a. Abb. 4):

### Einführung des Hexamethylen-Spacers:

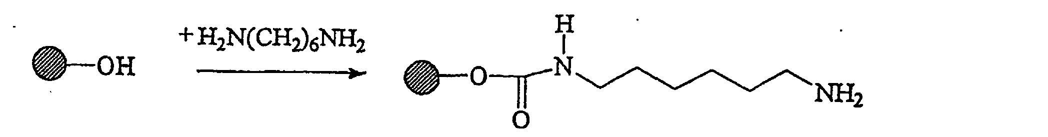

In 5 ml einer 0,3 M Lösung von 1,1'-Carbonyldiimidazol in DMF werden 180 mg im HV getrocknete hydroxylierte Polyolefin-Fritten (Darstellung s.o.) unter Stickstoff 6 Stunden bei RT geschüttelt. Danach werden die Fritten 3 x mit je 50 ml DMF gespült und danach in 5 ml einer 0,3 M Lösung von 1,6-Diaminohexan in DMF 18 Stunden geschüttelt. Anschließend werden die Fritten mit DMF, Methanol, Aceton und Ether gewaschen und im HV bei RT getrocknet.

### Kopplung des Startnucleosids:

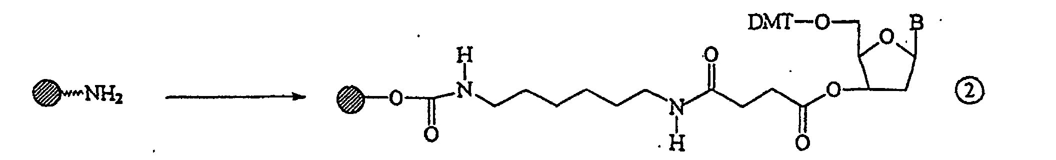

Durchführung analog Beispiel 1, jedoch ohne Methylimidazol.

### Funktionalisierung von PTFE

PTFE-Fritten werden mit Natriumnaphthalid behandelt und anschließend hydrolysiert. Bereits hier erhält man hydroxylierte PTFE-Fritten. Durch eine anschließende Hydroborierung kann man die Konzentration von Hydroxygruppen auf dem Träger noch erheblich steigern.

### Weiteres Trägermaterial (im PRIME96 getestet)

- Cellulose
- kommerzielles Trägermaterial (Fa. Pharmacia) als Pulver zwischen zwei Fritten

### E) Beispiele für Linker

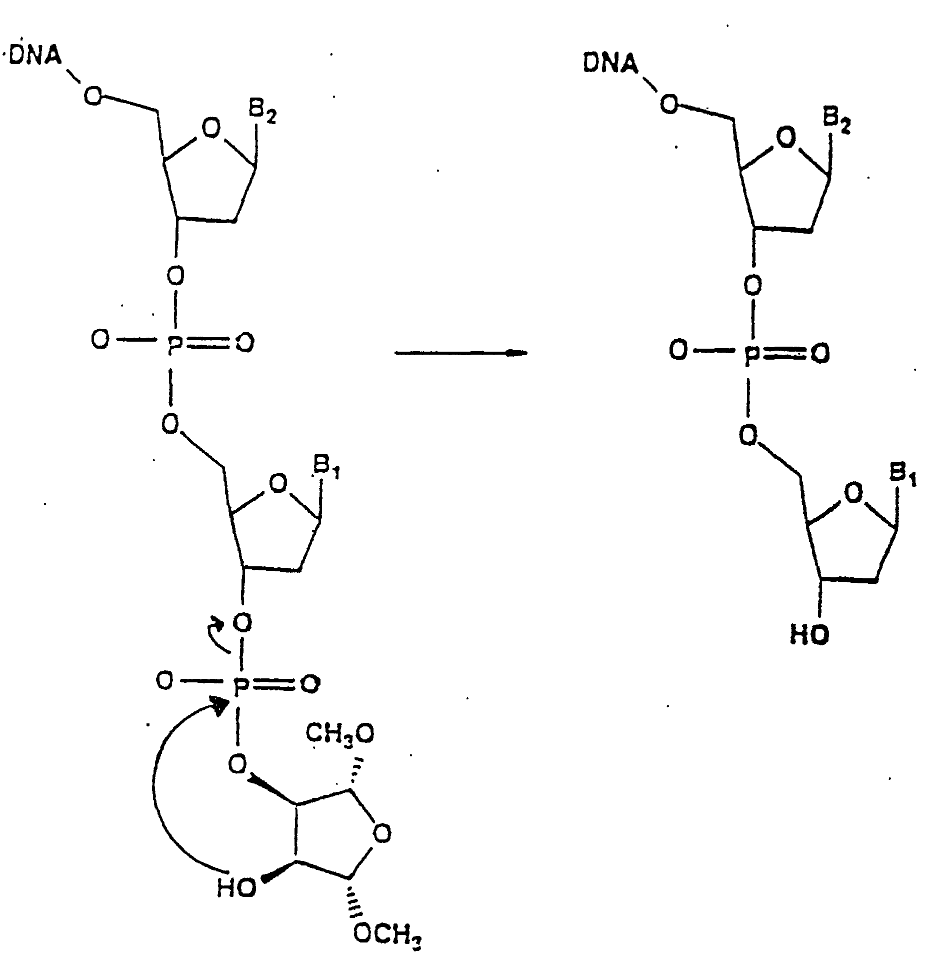

H. Köster und K. Heyns (1972)^, Tetrahedron Letters 1972, 1531
G.R. Gough, M.J. Brunden und P.T. Gilham (1983), Tetrahedron Letters 1983, 5321
Scott, P. Hardy, R.C. Sheppard und M.J. McLean (1994); in: Solid Phase Synthesis (R. Epton, Hg.), Mayflower Worldwide Ltd., Birmingham, UK, S. 115

### F) Beispiele für poröse Frittenböden

### Reaktionsschema:

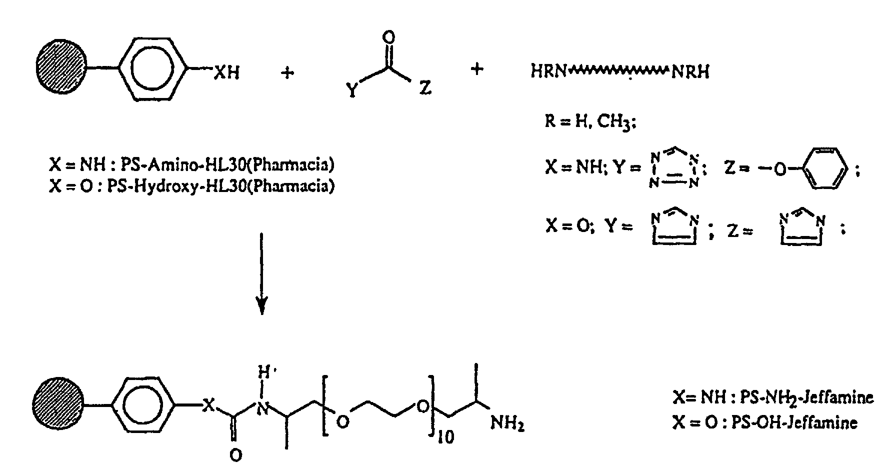

| **Name** | **-X** | **Träger- material** | **Versuchs- nummer** | **Beladungs- nummer** | **Capping** | **Beladungs- zahl** |
|---|---|---|---|---|---|---|
| PS-NH₂-Jeffamine | -NH | PS-Amino-HL30 | -- | V224A | -- | -- |
| Bedingungen: 106mg (15,2µmol)Träger; 18,5mg (97,9µmol) PCT | | | | | | |
| Aktivierung: 18h/RT/1ml AcN); 1,75mmol Jeffamine 500; 24h/RT+5h/60°C | | | | | | |
| PS-NH₂-Jeffamine | -NH | PS-Amino-HL30 | -- | V232A | -- | -- |
| Bedingungen: 510mg (73µmol)Träger; 163mg (862µmol) PCT | | | | | | |
| Aktivierung: 17h/RT/5ml AcN; 0,525mmol Jeffamine 500; 2,5h/RT+5,5h/60°C | | | | | | |
| PS-NH₂-Jeffamine | -NH | PS-Amino-HL30 | -- | V288B3 | | BPB: 60µmol/g |
| Bedingungen: 1,024g (145µmol)Träger; 200mg (1,053mmol) | | | | | | |
| Aktivierung: 24h/RT/20ml AcN);400mg akt. Träger+300µl Jeffamine 500/24h/60°C1 | | | | | | |
| Capping: 10ml DMF; 3ml Pyridin; 0,5ml Ac₂O; 15min/RT; | | | | | | |
| PS-OH-Jeffamine | -O | PS-Hydroxy-HL30 | -- | V232B | -- | BPB: 72µmol/g |
| Bedingungen: 357mg Träger; 137mg CDI (844µmol) | | | | | | |
| Aktivierung: 17h/RT/5ml AcN; 525µmol Jeffamine 500; 2,5h RT+5,5h/60°C | | | | | | |

### Vorteile des Spacermoleküls:

- Nach Aktivierung der Aminofunktion können die nicht umgesetzten Funktionen auf der Trägeroberfläche mit Essigsäureanhydrid gecappt werden. Mögliche Nebenreaktionen durch vicinale Hydroxyfunktionen (intramolekularer Ringschluß und Abspaltung vom Träger während der Ammoniakbehandlung) werden dadurch vermieden.
- Die Beladung kann durch Anfärben der nicht abreagierten basischen Aminofunktionen über den Bromphenolblau(BPB)-Test nach Aktivierung, Capping bzw. Aminolyse ermittelt werden.
- Durch die Verwendung von bisfunktionellen Aminopolyglykolspacern (z.B. Jeffamin 500) wird die hydrophobe Trägeroberfläche hydrophiler. Dies kann sich positiv auf die Reaktionsausbeuten bei der Oligomerensynthese auswirken (z.B. verringert sich die elektrostatische Aufladung, die hydrophilen Spacerarme ragen weiter in polare Lösungsmittel hinaus etc.)

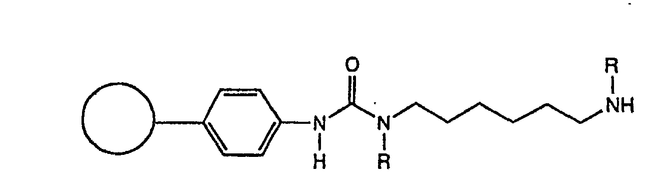

| **Name** | **-X (-R)** | **Träger- material** | **Versuchs- Nummer** | **Beladungs- nummer** | **Capping** | **Beladungs- zahl** |
|---|---|---|---|---|---|---|
| PS-NH₂-Amino-hexane | -NH | PS-Amino-HL30 | -- | 277 | | BPB: 102µmol/g |
| | (-H) | | | | | |
| Bedingungen: 500mg (71,5µmol)Träger; 50mg PCT (264µmol); 24h/RT;600µmol 1,6-Diaminohexane 4,0ml AcN/DMF(1/1) 60°C/12h | | | | | | |
| Capping: Ac₂O DMF Pyridin (0,5/5/2ml) 500mg DMAP 10 min/RT | | | | | | |
| PS-NH₂-Aminohexane | -NH | PS-Amino-HL30 | -- | V288.B4 | -- | BPB: 188µmol/g (?) |
| | (-H) | | | | | |
| Bedingungen: 1,024g (144µmol)Träger; 200mg PCI (1053µmol); 24h/RT; 600 mg alkt. Träger+420mg 1,6-Diaminohexane 12h/60°C | | | | | | |
| PS-NH₂-Methylaminohexane | -NH | PS-Amino-HL30 | -- | V312 | | BPB: 97µmol/g |
| Bedingungen: 1,031g (145µmol)Träger; 125mg PCT (0,661mmol); | | | | | | |
| Aktivierung: 3h/RT/10ml AcN; 300mg N,N'-Dimethyl-1,6-hexanediamine; 16h/60°C | | | | | | |
| Capping: Ac₂O DMF Pyridin (0,5/5/2ml) 500mg DMAP 15 min/RT | | | | | | |

### Reaktionsschema:

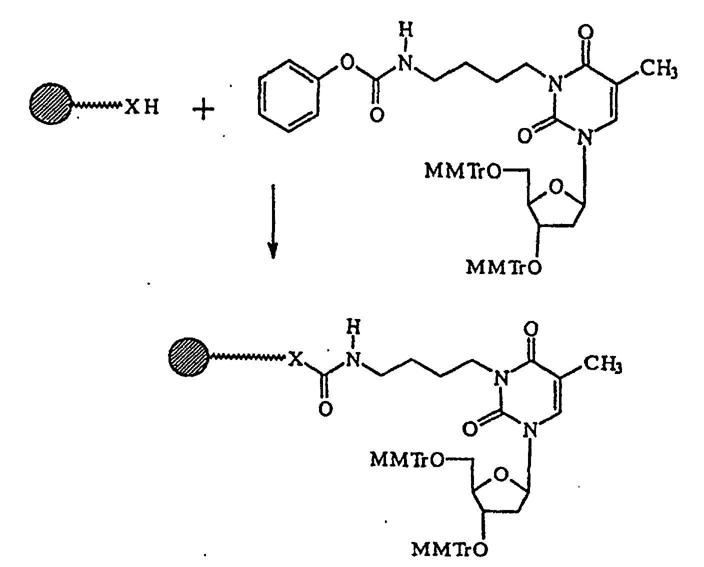

| **Name** | **-X** | **Träger- material** | **Versuchs- Nummer (Spacer)** | **Beladungs- nummer** | **Capping** | **Beladungs- zahl** |
|---|---|---|---|---|---|---|
| PS-NH₂-Thymidinhexane-3',5'-MMTr | -NH | PS-Amino-HL30 | V210 | V225 | | MMTr: 27µmol/g |
| Bedingungen: 118mg (17µmol)Träger; 8,2mg (8,4µmol); 15h/60°C/1,5ml AcN | | | | | | |
| Capping: Ac₂O DMF Pyridin (0,1/1,0/0,5ml) 100mg DMAP 60 min/RT | | | | | | |
| PS-NH₂-Jeffamine -Thymidinhexane-3',5'-MMTr | NH | PS-NH₂-Jeffamine | V210/V232A | V240A | -- | MMTr: 15µmol |
| Bedingungen: 86mg V232A 7,8mg (8,0µmol) V210; 25h/60°C/1,0ml AcN | | | | | | |
| PS-OH₂-Jeffamine -Thymidinhexane-3',5'-MMTr | O | PS-OH₂-Jeffamine -Thymidinhexane-3',5'-MMTr | V210/V232B | V240B | -- | MMTr: 18µmol |
| Bedingungen: 60mg V232B 7,7mg (7,9µmol)V210; 25h/60°C/1,0ml AcN | | | | | | |

### Reaktionsschema:

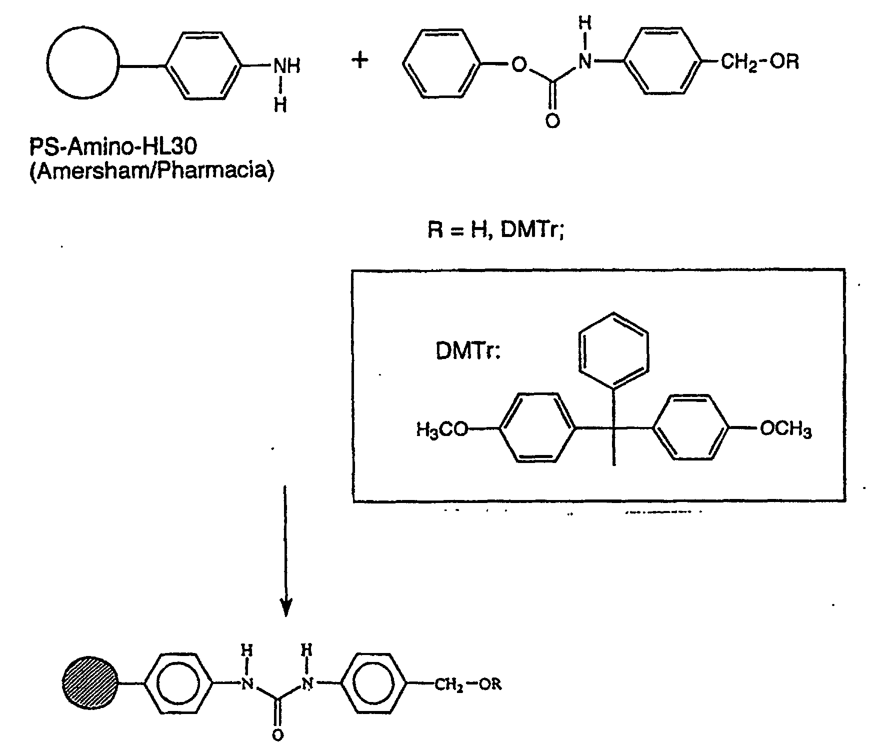

| **Name** | **-R** | **Träger- material** | **Versuchs- nummer** | **Beladungs- Nummer** | **Capping** | **Beladungs- zahl** |
|---|---|---|---|---|---|---|
| PS-NH₂-Benzyl-alkohol-OH | -H | PS-Amino-HL30 | D744 | 310 | | Indirekt über BPB 110 µmol/g |
| Bedingungen: 500mg (71,5µmol)Träger; 80mg (329µmol=4,6 Moläquivalente) 20h/60-70°C in 5ml CH₃CN | | | | | | |
| Capping: DMAP Ac₂O CH₃CN 30min.RT | | | | | | |
| PS-NH₂-Benzylalkohol-ODMTr | -DMTr | PS-Amino-HL30 | V366-P1 | -- | | |
| Bedingungen: | | | | | | |

### Vorteile des Spacermoleküls:

- Eine mögliche Nebenreaktion durch vicinale Hydroxyfunktionen wird vermieden.
- Die Beladung kann durch Anfärben der nicht abreagierten basischen Aminofunktionen über den Bromphenolblau(BPB)-Test ermittelt werden, insbesondere wenn R = H. Bei der tritylierten Variante kann die Beladung entweder über BPB oder nach dem Cappen über den Tritylwert bestimmt werden.
- Bei Beladung mit einem 3'-O-Phosphoramidit entstehen statt der säurelabilen Phosphorsäureamidate die stabileren Phosphorsäurediester (W. Bannwarth; Helv. Chim. Acta, Band 71, 1517 (1988).

### Bezugszeichenliste

- 1.: Pipettierrobotter
- 2.: Reagenz
- 3.: Baustein
- 4.: Reaktionsgefäß / Reaktor / Reaktionssäule
- 5.: Trägermaterial / Syntheseträger
- 6, 6': Frittenboden (Deckfritte)
- 7.: Wanne
- 8, 8': Ventile
- 9.: Reaktorblock (Reaktionsblock)
- 10.: Lochblende (Prallblech)
- 11.: Vakuumpumpe
- 12.: Lösungsmittel (Solvensflasche)
- 13.: Dosiernadel / Mehrfachkanüle
- 14, 14', 14'': Nadelkanal
- 15, 15', 14'': Dosierspritze (Dilutor)
- 16.: Transferport
- 17.: Vorratsflasche
- 18.: schaltbares Ventil
- 19.: Dosierkamm
- 20.: Auffanggefäß / Mikrofilterplatte
- 21.: Block für Reagenzien und Bausteine
- 22.: Gefäß
- 23.: Roboter
- 24.: Solvens 1, 2, 3
- 25.: Abfallflasche
- 26.: Stickstoff
- 27.: Inertgas / Schutzgas
- 28.: Feder
- 29.: Anordnung für Abspaltung und Aufreinigung

## Patentansprüche

1. Vorrichtung für eine automatisierte simultane chemische Synthese und fakultative Aufreinigung einer Vielzahl von Produkten an der Festphase, die eine Vielzahl von separaten, nach oben und unten offenen Reaktionsgefäßen bzw. Reaktoren als parallel in einem Block bzw. Reaktorblock angeordnete Kanäle bzw. Reaktionskanäle oder Säulen bzw. Reaktionssäulen aufweist, die entweder gemeinsam oder einzeln entnehmbar sind; wobei in den Kanälen bzw. Säulen Trägermaterial für die Synthese, d. h. eine Festphase, entweder zwischen zwei inerten porösen Frittenböden oder selbst als chemisch modifizierte Fritten- oder Filterböden eingebracht ist, so daß von oben zugegebene flüssige Medien allein durch die Oberflächenspannung und Benetzung des Trägermaterials im Reaktor bzw. in den Reaktionsgefäßen festgehalten werden können; und der Block bzw. Reaktorblock auf eine Wanne aufgesetzt ist, die über ein schaltbares Ventil an eine Vakuumpumpe angeschlossen ist, so daß flüssige Medien aus den Reaktionsgefäßen bzw. Reaktoren und den darin enthaltenen Trägermateralien simultan abgesaugt werden können, **dadurch gekennzeichnet, daß** die oberen Eingänge der Säulen bzw. Reaktionssäulen im Reaktorblock durch eine darüber angebrachte Lochblende oder ein darüber angebrachtes Prallblech abgedeckt sind, so daß die Reaktionssäulen mit einem Inertgas geflutet werden können und der Inertgasstrom gegebenenfalls während des Absaugvorganges deutlich erhöht werden kann; oder daß der Raum über den Reaktionssäulen bzw. Reaktionskanälen durch eine verschiebbare Lochblende gezielt verschlossen werden kann, so daß die Reagenzien mit Inertgasüberdruck aus den Reaktionssäulen oder Reaktionskanälen ausgeblasen werden können.

2. Vorrichtung nach Anspruch 1, **dadurch gekennzeichnet, daß** sie mit einer Inertgasversorgung versehen ist, insbesondere für Stickstoff oder Argon.

3. Vorrichtung nach Anspruch 1 oder 2, **dadurch gekennzeichnet, daß** die Vorrichtung mit einem xyz-Pipettierroboter, mit elektronisch steuerbaren Dosierspritzen bzw. Dilutoren mit einer oder mit mehreren Dosiernadeln und gegebenenfalls zusätzlich mit einem oder mit mehreren Dosierkämmen versehen ist, so daß chemische Bausteine, Reagenzien und Lösungsmittel auf die Reaktionsgefäße verteilt werden können und jeder Reaktor einzeln adressierbar ist.

4. Vorrichtung nach Anspruch 3, **dadurch gekennzeichnet, daß** jede Dosiernadel mit mehreren, mindestens mit zwei, an separate Dosierspritzen angeschlossenen und damit getrennt befüllbaren inneren Kanälen versehen ist, deren Enden sich erst kurz vor dem Auslaß treffen, so daß bei simultaner Dosierung mehrerer Reagenzien das Mischen erst kurz vor der Abgabe in der Spitze der Dosiernadel erfolgt; wobei gegebenenfalls ein Kanal auch an die Inertgasversorgung angeschlossen sein kann, so daß über ein Inertgaspuls das Mischvolumen ausgedrückt werden kann.

5. Vorrichtung nach Anspruch 3 oder 4, **dadurch gekennzeichnet, daß** die Dosiernadeln in der Längsachse federnd gelagert sind, so daß die Dosiernadeln auf das Trägermaterial oder die Deckfritten in den Reaktorkanälen aufgesetzt und so auch kleinste Volumina bis herunter zu 1 Nanoliter sicher abgesetzt werden können.

6. Vorrichtung nach einem der vorhergehenden Ansprüche, **gekennzeichnet durch** mit Septen verschlossene Gefäße, die in einem vom Reaktionsblock getrennten Reagenzienblock für eine Vielzahl, insbesondere 2 bis 100 und vorzugsweise 24, chemische Bausteine und Reagenzien vorgesehen sind.

7. Vorrichtung nach Anspruch 6, **dadurch gekennzeichnet, daß** die Hälse der Gefäße im Reaktorblock mit Septen verschlossen und durch eine darüber angebrachte Lochblende oder ein Prallblech, abgedeckt sind, so daß sie mit Inertgas, vorzugsweise mit Stickstoff oder Argon, geflutet werden können.

8. Vorrichtung nach Anspruch 3 oder 4, **gekennzeichnet durch** Transferports, die entweder direkt oder über schaltbare Ventile mit Vorratsgefäßen bzw. Vorratsflaschen verbunden sind, wobei diese Vorratsgefäße gegebenenfalls mit einem geringen Überdruck beaufschlagbar sind, so daß Reagenzien mit Dosiernadeln auch aus Transferports entnommen werden können.

9. Vorrichtung nach einem der vorhergehenden Ansprüche, insbesondere nach Anspruch 3, **gekennzeichnet durch** Solventflaschen, Dosierspritzen und ein oder mehrere Dosierkämme, so daß Lösungsmittel und/oder Reagenzien aus Solventflaschen mittels Dosierspritzen oder **durch** Inertgasüberdruck auch über einen oder mehrere Dosierkämme simultan auf mehrere Reaktoren reihenweise verteilt werden können.

10. Vorrichtung nach einem der vorhergehenden Ansprüche, insbesondere nach Anspruch 1 oder 2, **dadurch gekennzeichnet, daß** das Trägermaterial eine Schicht im Reaktorkanal bildet, die gleichmäßig von aufgegebenen Reagenzien und/oder Lösungsmitteln allein durch die Schwerkraft durchströmt werden kann.

11. Vorrichtung nach einem der vorhergehenden Ansprüche, **gekennzeichnet durch** einen Steuerungsrechner, der die Gesamtheit aller Produkte für ein Syntheseprogramm mit Hilfe einer Liste von ASCII-Worten einer Software, bei der die chemischen Bausteine bzw. Monomeren, die für den Aufbau der Produkte eingesetzt werden, als ASCII-Letter codiert sind und die Produkte so als Abfolge von Aufbaureaktionen bzw. Monomereinbaureaktionen **durch** ASCII-Worte beschrieben sind, in Ventilschaltungs-, Dosierspritzenbewegungs- und Roboterarmbewegungsoperationen umsetzen kann, wobei jeder Monomereinbau aus einer Folge von mehreren Reaktionsschritten und Schaltungsoperationen bestehen kann.

12. Reaktor nach einem der vorhergehenden Ansprüche, **gekennzeichnet durch** eine zum Reaktor bzw. zur Reaktoranordnung komplementäre Anordnung von Affinitätssäulen und/oder eine der Reaktoranordnung komplementäre Anordnung von Auffanggefäßen.

## Claims

1. Apparatus for automated simultaneous chemical synthesis and optional purification of a large number of products on solid phase, which apparatus has a large number of separate reaction vessels/reactors, which are open at the top and at the bottom, in the form of channels/reaction channels or columns/reaction columns which are arranged in parallel in a block/reactor block and which are removable either together or separately; wherein support material for the synthesis, that is to say a solid phase, is placed in the channels/columns, either being arranged between two inert porous frit plates or being itself in the form of chemically modified frit or filter plates, so that liquid media added from above can be held in the reactor/reaction vessels solely as a result of surface tension and wetting of the support material and the block/reactor block is mounted on a trough connected to a vacuum pump by means of a switchable valve so that liquid media can be aspirated simultaneously from the reaction vessels/reactors and the support materials contained therein, **characterised in that** the upper inlets to the columns/reaction columns in the reactor block are covered by a perforated screen mounted above them or a baffle plate mounted above them, so that the reaction columns can be flooded with an inert gas and the flow of inert gas can optionally be increased considerably during the aspirating procedure; or the space above the reaction columns/reaction channels can be selectively closed off by means of a displaceable perforated screen so that the reagents can be blown out of the reaction columns or reaction channels by pressurised inert gas.

2. Apparatus according to claim 1, **characterised in that** it is provided with an inert gas supply, especially for nitrogen or argon.

3. Apparatus according to claim 1 or 2, **characterised in that** the apparatus is provided with an xyz-pipetting robot, having electronically controllable dispensing syringes/ dilutors having one or more dispensing needles and optionally, in addition, having one or more dispensing manifolds, so that chemical building blocks, reagents and solvents can be distributed to the reaction vessels and each reactor can be addressed individually.

4. Apparatus according to claim 3, **characterised in that** each dispensing needle is equipped with a plurality of, at least two, internal channels, which are connected to separate dispensing syringes and which consequently can be filled separately, the ends of which channels meet only shortly before the outlet, so that, when a plurality of reagents are being dispensed simultaneously, mixing occurs only shortly before delivery in the tip of the dispensing needle; a channel optionally being connected to the inert gas supply also, so that a pulse of inert gas can expel the mixed volume.

5. Apparatus according to claim 3 or 4, **characterised in that** the dispensing needles are mounted so as to be resilient along the longitudinal axis, so that they can be set down on the support material or top frits in the reactor channels, and so even extremely small volumes down to 1 nanolitre can be reliably deposited.

6. Apparatus according to any one of the preceding claims, **characterised by** vessels which are sealed by means of septa and provided in a reagent block separate from the reaction block for a large number, especially from 2 to 100 and preferably 24, of chemical building blocks and reagents.

7. Apparatus according to claim 6, **characterised in that** the necks of the vessels in the reactor block are sealed by means of septa and covered by a perforated screen or a baffle plate, mounted above them, so that they can be flooded with inert gas, preferably nitrogen or argon.

8. Apparatus according to claim 3 or 4, **characterised by** transfer ports, which are connected to storage vessels/storage bottles either directly or by means of switchable valves, those storage vessels optionally being arranged to be slightly pressurised, so that reagents can also be withdrawn from transfer ports by means of dispensing needles.

9. Apparatus according to any one of the preceding claims, especially according to claim 3, **characterised by** solvent bottles, dispensing syringes and one or more dispensing manifolds, so that solvents and/or reagents can be distributed from solvent bottles by means of dispensing syringes or by pressurised inert gas and also by way of one or more dispensing manifolds simultaneously to a plurality of reactors row by row.

10. Apparatus according to any one of the preceding claims, especially according to claim 1 or 2, **characterised in that** the support material forms a layer in the reactor channel, through which an even flow of applied reagents and/or solvents can pass solely under the action of gravity.

11. Apparatus according to any one of the preceding claims, **characterised by** a control computer, by means of which it is possible, using a list of ASCII words of software wherein the chemical building blocks/monomers used for building up the products are coded as ASCII characters and the products are therefore described as a sequence of build-up reactions/monomer incorporation reactions by means of ASCII words, for the totality of all products for a synthesis program to be converted into valve-switching operations, dispensing-syringe movement operations and robot arm movement operations, it being possible for each monomer incorporation to consist of a succession of several reaction steps and switching operations.

12. Reactor according to any one of the preceding claims, **characterised by** an arrangement of affinity columns which is complementary to the reactor/ reactor arrangement and/or an arrangement of collection vessels which is complementary to the reactor arrangement.

## Revendications

1. Dispositif pour une synthèse chimique et une purification facultative automatisées simultanées d'une pluralité de produits sur une phase solide, comportant une pluralité de récipients réactionnels ou réacteurs distincts, ouverts vers le haut et vers le bas, sous forme de canaux/canaux réactionnels ou de colonnes/colonnes réactionnelles, qui sont disposés parallèlement les uns aux autres dans un bloc ou un bloc réactionnel et peuvent être retirés séparément ou conjointement, les canaux ou colonnes contenant un support pour la synthèse, c'est-à-dire une phase solide disposée entre deux plaques de verre fritté poreux inerte ou se présentant elle-même sous la forme de plaques de verre fritté ou de plaques de filtre modifiées par des fonctions chimiques, de manière à ce que des phases liquides introduites par le haut puissent être retenues dans le réacteur ou les récipients réactionnels par la seule tension superficielle et le mouillage du support, le bloc ou le bloc réactionnel étant disposé sur une cuve qui est reliée, via une vanne commutable, à une pompe à vide, afin que les phases liquides provenant des récipients réactionnels ou des réacteurs et des supports contenus dans ceux-ci, puissent être aspirées simultanément, **caractérisé par le fait que** les entrées supérieures des colonnes/colonnes réactionnelles dans le bloc réactionnel sont couvertes par une plaque perforée ou une chicane de manière à ce que les colonnes réactionnelles puissent être parcourues par un flux de gaz inerte et que le cas échéant le flux de gaz inerte puisse être augmenté nettement pendant la phase d'aspiration, ou **par le fait que** l'espace au dessus des colonnes réactionnelles ou des canaux réactionnels peut être fermé de manière ciblée au moyen d'une plaque perforée mobile, de manière à ce que les réactifs puissent être expulsés des colonnes réactionnelles ou des canaux réactionnels grâce à une surpression de gaz inerte.

2. Dispositif selon la revendication 1, **caractérisé par le fait qu'**il est pourvu d'une alimentation en gaz inerte, en particulier en azote ou argon.

3. Dispositif selon la revendication 1 ou 2, **caractérisé par le fait qu'**il est pourvu d'un robot de pipetage-xyz, de seringues de dosage ou de dilution à commande électronique, avec une ou plusieurs pointes-doseuses, et en outre éventuellement d'un ou de plusieurs peignes-doseurs, de telle sorte de répartir des pièces chimiques, des réactifs et des solvants entre les récipients réactionnels et d'adresser chaque réacteur séparément.

4. Dispositif selon la revendication 3, **caractérisé par le fait que** chaque pointe-doseuse est pourvue de plusieurs, au moins deux, canaux intérieurs, qui sont reliés à des seringues de dosage séparées et peuvent de ce fait être remplis indépendamment, et dont les extrémités ne se rejoignent qu'immédiatement avant la sortie, de sorte que dans le cas d'un dosage simultané de plusieurs réactifs, le mélange de ceux-ci a lieu juste avant l'injection au niveau de la pointe de la seringue de dosage, l'un des canaux pouvant éventuellement être relié à l'alimentation de gaz inerte, une impulsion de gaz inerte permettant alors d'expulser le volume du mélange.

5. Dispositif selon la revendication 3 ou 4, **caractérisé par le fait que** les pointes-doseuses sont montées élastiquement dans la direction longitudinale, de sorte que les pointes-doseuses peuvent être appliquées sur le support ou sur le verre fritté de couverture dans les canaux réactionnels et qu'ainsi même de très faibles volumes, jusqu'à 1 nanolitre, peuvent être déposés de manière sûre.

6. Dispositif selon une des revendications précédentes, **caractérisé par** des récipients fermés par des septums, qui sont prévus dans un bloc à réactifs distinct du bloc réactionnel, pour une pluralité, en particulier pour 2 à 100 et de préférence pour 24 pièces chimiques et réactifs.

7. Dispositif selon la revendication 6, **caractérisé par le fait que** les cols des récipients dans le bloc réactionnel sont fermés par des septums et sont couverts par une plaque perforée ou une chicane placée au-dessus, de manière à permettre la circulation d'un gaz inerte, de préférence de l'azote ou de l'argon.

8. Dispositif selon la revendication 3 ou 4, **caractérisé par** des orifices de transfert qui sont connectés, soit de manière directe, soit par l'intermédiaire de vannes commutables à des récipients de stockage ou à des bouteilles de stockage, une légère surpression pouvant le cas échéant être appliquée auxdits récipients de stockage de manière à ce que des réactifs puissent être prélevés également au niveau des orifices de transfert à l'aide de pointes-doseuses.

9. Dispositif selon une des revendications précédentes, notamment selon la revendication 3, **caractérisé par** des bouteilles de solvants, des seringues de dosage et un ou plusieurs peignes-doseurs, de sorte que des solvants et/ou des réactifs de bouteilles à solvants peuvent être distribués de manière simultanée, en ligne, dans plusieurs réacteurs à l'aide de seringues de dosage ou par une surpression de gaz inerte, ou encore à l'aide d'un ou de plusieurs peignes-doseurs.

10. Dispositif selon une des revendications précédentes, notamment selon la revendication 1 ou 2, **caractérisé par le fait que** le support, dans le canal réactionnel, forme une couche qui peut être traversée de manière régulière, uniquement par gravité, par des réactifs et/ou des solvants.

11. Dispositif selon une des revendications précédentes, **caractérisé par** un calculateur de commande qui convertit en opérations de commutation de vanne, de déplacement de seringue de dosage et de déplacement de bras de robot, l'ensemble des produits pour un programme de synthèse, au moyen d'une liste de mots ASCII d'un logiciel, dans laquelle les pièces chimiques et les monomères utilisés pour la synthèse des produits sont codés sous forme de caractères ASCII et les produits, en tant que suites de réactions de synthèse ou de réactions de greffage de monomères sont décrits par des mots ASCII, chaque greffage de monomère pouvant être formé d'une suite de plusieurs étapes de réaction et d'opérations de commutation.

12. Réacteur selon une des revendications précédentes, **caractérisé par** un ensemble complémentaire du réacteur ou du système de réacteurs, formé de colonnes d'affinité et/ou un ensemble de récipients de collecte complémentaire du système de réacteurs.
